(19) Europäisches Patentamt
European Patent Office
Office européen des brevets

(11) Veröffentlichungsnummer: **0 233 206**
**B1**

(12) # EUROPÄISCHE PATENTSCHRIFT

(45) Veröffentlichungstag der Patentschrifft: **21.03.90**

(51) Int. Cl.⁵: **C 07 K 9/00**, A 61 K 37/02, A 61 K 39/39, A 61 K 31/01 // C07H9/04, C07H15/04

(21) Anmeldenummer: **86904104.6**

(22) Anmeldetag: **17.07.86**

(86) Internationale Anmeldenummer:
**PCT/DE 86/00295**

(87) Internationale Veröffentlichungsnummer:
**WO 87/00529 (29.01.87 Gazette 87/03)**

(54) **ZUCKERHALTIGE DIPEPTIDE UND IHRE PHARMAZEUTISCHE ANWENDUNG.**

(30) Priorität: **27.07.85 DE 3526922**

(43) Veröffentlichungstag der Anmeldung:
**26.08.87 Patentblatt 87/35**

(45) Bekanntmachung des Hinweises auf die Patenterteilung:
**21.03.90 Patentblatt 90/12**

(84) Bennante Vertragsstaaten:
**AT BE CH DE FR GB IT LI LU NL SE**

(56) Entgegenhaltungen:
**EP-A-0 118 364**
**EP-A-0 143 746**

**Carbohydrate Research, volume 94, 1981 Elsevier Scientific Publishing Co. Amsterdam (NL) A. Hasegava et al.: "Synthesis of carbohydrate analogs (positional configurational, and optical) of N-acetylmuramoyl-L-alanyl-D-isoglutamine, and their immunoadjuvant activities", pages 143-163, see pages 143, 144; page 148: formula 47**

**Chemical Abstracts, vol. 99, No. 7, 15 August 1983, Columbus, Ohio (US) - T. Yoshiro et al.:" Regression of line-10 hepatocarcinoma with synthetic quinonyl muramyl dipeptide in strain-2 guinea pigs" see page 24, abstract 47616q, & Gann 1983, 74(2), 192-5 (Eng.)**

**Die Akte enthält technische Angaben, die nach dem Eingang der Anmeldung eingereicht wurden und die nicht in dieser Patentschrift enthalten sind.**

(73) Patentinhaber: **STADA ARZNEIMITTEL AG**
**D-6368 Bad Vilbel (DE)**

(72) Erfinder: **Bradaczek, Hans**
**Bergengruenstrasse 47**
**D-1000 Berlin 38 (DE)**
Erfinder: **Masihi, Kemuel Noel**
**Pannierstrasse 25**
**D-1000 Berlin 33 (DE)**
Erfinder: **Labischinski, Harald**
**Koenigsallee 42 A**
**D-1000 Berlin 33 (DE)**
Erfinder: **Gruszecki, Wojciech**
**Berlepschstrasse 132**
**D-1000 Berlin 37 (DE)**

(74) Vertreter: **von Kreisler, Alek, Dipl.-Chem.**
**Patentanwälte Von Kreisler-Selting-Werner**
**Deichmannhaus am Hauptbahnhof**
**D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBERGRAF, STOCKHOLM 1990

**Beschreibung**

Die Erfindung betrifft neuartige, zuckerhaltige Dipeptide in furanoider Form, deren Herstellung und ihre Anwendung für verschiedene pharmazeutische, biologische und medizinische Anwendungsgebiete insbesonders im Rahmen von Infektionsprophylaxe. Bekämpfung von Infektionskrankheiten verschiedenen Ursprungs, von Tumoren und vergleichbaren Krankheitsbildern, zur Verwendung als Zusatzstoff in verschiedenen z. B. pharmazeutischen Erzeugnissen und Impfstoffen sowie als Adjuvans oder Modulator der Immunantwort.

Nachdem 1974 F. Ellouz et al. die Immunostimulation von Bakterienzellwand-Fragmenten in der Form von N-acetyl-muramyl-L-alanyl-D-isoglutamin (MDP = Muramyldipeptid) [Biochem. Biophys. Res. Commun. 59, 1317 (1974)] entdeckten, entstanden eine Vielzahl von Publikationen und Patenten, die über die biologische Wirkung von dieser Art von Substanzen berichteten. MDP ist ein in pyranoider Form vorliegendes Zuckerhaltiges Dipeptid.

Infolge ihrer Wirkung z. B. über eine Erhöhung der Menge von Antikörpern im lebenden Organismus sollten diese Substanzen durchweg mehr oder weniger aktiv z. B. gegen Virusund Bakterien- und andere Infektionen, aber z. B. auch gegen Tumorerkrankungen sein, wie zum Teil auch durch in vitro-Tests gezeigt werden konnte. Bei in vivo-Tests allerdings zeigten diese Substansen für sich kaum Schutzwirkungen. In einigen Fällen kann diese Schutzwirkung durch Beifügungen von Zusatzstoffen – z. B. öligen Mycolaten hervorgerufen werden; allerdings führen diese Zusatzstoffe zu unerwünschten Nebenwirkungen, wie toxische Effekte, Fieber u. a. [J. Biol. Resp. Modif., Vol. 3, No. 6, 663 - 671 (1984)].

A. Hasegawa et al beschreiben in "Carbohydrate Research", 94 (1981), 143 – 163, verschiedene zuckerhaltige Dipeptide. Mit diesen Verbindungen wird die Frage untersucht, welchen Einfluß bestimmte strukturelle Variationen in der Kohlenstoffseitenkette auf die immunstimulierende Wirkung dieser Verbindungen ausüben. Die immunstimulierende Wirkung der zum N-Acetylmuramoyldipeptid analogen Verbindungen wurde am Meerschweinchen untersucht. Daten zur immunstimulierenden Wirkung dieser Analoga wurden ausschließlich an pyranoiden Zuckerdipeptiden ermittelt. Als Zwischenverbindungen zur Synthese der pyranoiden aktiven Zuckerdipeptide werden auch furanoide Derivate beschrieben, die jedoch auf eine immunstimulatorische Wirkung nicht untersucht worden sind.

Der Erfindung liegt daher die Aufgabe zugrunde, neuartige Wirkstoffe und Methoden zu ihrer Herstellung zu entwickeln, deren Anwendung zugleich die oben erwähnten Nachteile und Beschränkungen des Standes der Technik überwindet.

Die Erfindung betrifft zuckerhaltige Dipeptide der Formel (I)

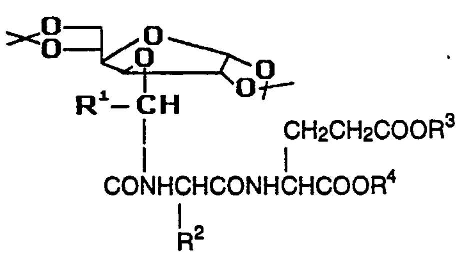

(I)

in der

$R^1$ – Wasserstoff oder eine $C_1$-$C_6$-Alkylgruppe

-HN-CH-CO-
|
$R^2$
– Glycin oder einen natürlichen L-Aminosäurerest

$R^3$ – Wasserstoff oder eine $C_1$-$C_6$-Alkylgruppe und

$R^4$ – Wasserstoff oder eine $C_1$-$C_6$-Alkylgruppe

bedeuten, und der Glutamylrest in der D-Form vorliegt.

Überraschenderweise sind die erfindungsgemäßen furanoiden Zuckerhaltigen Dipeptide in in vivo-Tests mit und ohne Beifügungen biologisch sehr aktiv, so daß keine unerwünschten Nebenwirkungen zu erwarten sind. Mit ihrer Hilfe könnten gegebenenfalls sehr wirkungsvolle Arzneimittel zur Prophylaxe und Bekämpfung z. B. mikrobieller Krankheiten (Viren, Bakterien, Parasiten, Pilze), aber auch für andere Erkrankungen z. B. Tumore sowie für immunmodulatorische Zwecke z. B. auch als Adjuvans entwickelt werden.

Die Reste $R^1$, $R^3$ und $R^4$ in der Formel (I) stehen bevorzugt für Wasserstoff oder geradkettige oder verzweigte $C_1$-$C_4$-Alkylreste. Die Reste können gleich oder verschieden sein.

Ganz besonders bevorzugt stehen $R^1$, $R^3$ und $R^4$ für Wasserstoff, Methyl oder Ethyl.

Zuckerhaltige Dipeptide, in denen -HN-CH-CO in Formel (I)
|
$R^2$

sich von Glycin, L-Alanin, L-Phenylalanin, L-Serin, L-Cystein, L-Lysin, L-Histidin, L-Valin, L-Leucin, L-Isoleucin, L-Tryptophan, L-Asparagin, L-Threonin, L-Tyrosin, L-Methionin, L-Ornithin, L-Arginin, L-Prolin oder L-Hydroxyprolin ableitet, sind ebenfalls bevorzugt.

Insbesondere leitet sich -HN-CH-CO in Formel (I) von

$$\begin{array}{c}-HN-CH-CO\\ \quad | \\ \quad R^2\end{array}$$

Glycin, L-Alanin, L-Phenylalanin, L-Serin oder L-Threonin ab.

Ein weiterer Gegenstand der Erfindung ist ein Verfahren zur Herstellung von zuckerhaltigen Dipeptiden der Formel (I)

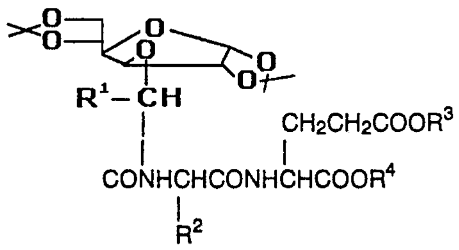

in der

| | |
|---|---|
| $R^1$ | – Wasserstoff oder eine $C_1$-$C_6$-Alkylgruppe |
| -HN-CH-CO- (mit $R^2$ an CH) | – Glycin oder einen natürlichen L-Aminosäurerest |
| $R^3$ | – Wasserstoff oder eine $C_1$-$C_6$-Alkylgruppe und |
| $R^4$ | – Wasserstoff oder eine $C_1$-$C_6$-Alkylgruppe |

bedeuten, und der Glutamylrest in der D-Form vorliegt, das dadurch gekennzeichnet ist, daß man D-Glucofuranosederivate der Formel (II)

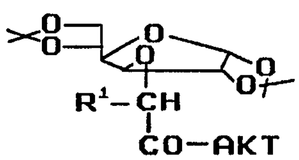

(II)

in der $R^1$ die oben angegebene Bedeutung hat und AKT für eine leicht auswechselbare, für Acylierungsreaktionen bekannte Gruppe steht, mit Dipeptidfragmenten der Formel (III)

$$\begin{array}{l}\quad\;\; R^2 \qquad\quad CH_2CH_2COOR^3\\ \quad\;\; | \qquad\qquad\quad |\\ H_2N\text{-}CHCONHCHCOOR^4\end{array} \qquad (III)$$

in der $R^2$, $R^3$ und $R^4$ die oben angegebene Bedeutung haben, zur Reaktion bringt und die Schutzgruppen in den Verbindungen der Formel (I) gegebenenfalls abspaltet.

Bevorzugt werden Verbindungen der Formel (II), die in einem ersten Schritt mit einer natürlichen L-Aminosäure umgesetzt, anschließend aktiviert und danach mit D-Glutaminsäure umgesetzt werden.

Weiterhin lassen sich die erfindungsgemäßen Verbindungen so herstellen, daß man Verbindungen der Formel (II) mit einem Carbonsäureimidchlorid der Formel (IV)

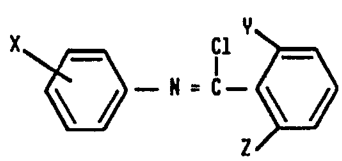

(IV)

in der

X Wasserstoff, $C_1$-$C_4$-Alkyl, Fluor, Chlor oder $NO_2$ bedeutet,
Y Halogen oder $NO_2$ darstellt und
Z die gleiche Bedeutung wie Y besitzt, umsetzt, das erhaltene Diacylamin mit der gewünschten L-Aminosäure zur Reaktion bringt, die so erhaltene Verbindung erneut mit einem Carbonsäureimidchlorid der Formel (IV) umsetzt und dieses Diacylamin mit D-Glutaminsäure zum Endprodukt der Formel (I) reagieren läßt.

Prinzipiell lassen sich die erfindungsgemäßen Verbindungen und deren Vorprodukte herstellen, indem man Verbindungen der Formel (II) mit einem Carbonsäureamidchlorid der Formel (IV)

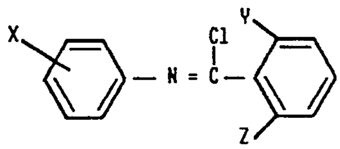

(IV)

in der

X Wasserstoff, $C_1$-$C_4$-Alkyl, Fluor, Chlor oder $NO_2$ bedeutet,
Y Halogen oder $NO_2$ darstellt und
X die gleiche Bedeutung wie Y besitzt,

umsetzt, das erhaltene Diacylamin mit der gewünschten L-Aminosäure oder Peptiden zur Reaktion bringt, die so erhaltene Verbindung erneut mit einem Carbonsäureimidchlorid der Formel (IV) umsetzt und dieses Diacylamin mit D-Glutaminsäure zum Endprodukt der Formel (I) reagieren läßt.

Das Verfahren wird in der Weise durchgeführt, daß man in einem ersten Reaktionsschritt eine Verbindung der allgemeinen Formel (II) (AKT = OH) mit einem Carbonsäureimidchlorid der allgemeinen Formel (IV) umsetzt. Dieser Reaktionsschritt wird vorzugsweise in einem inerten Lösungsmittel in Gegenwart von basischen Katalysatoren durchgeführt. Geeignete Lösungsmittel sind beispielsweise niedere Alkohole, wie Methanol, Ethanol oder Isopropanol, niedere Carbonsäureester, wie Ethylacetat, polare Ester, wie Dioxan, Tetrahydrofuran, Glykolmonomethylether oder Glykoldimethylether oder dipolare aprotische Lösungsmittel, wie Dimethylformamid, N-Methylacetamid, Sulfolan oder Hexamethylphosphorsäuretriamid. Geeignete Lösungsmittel sind ferner Gemische der obengenannten Lösungsmittel mit inerten unpolaren Lösungsmitteln, wie chlorierten Kohlenwasserstoffen (Dichlormethan, Trichlormethan oder Tetrachlorethan etc.) oder aromatische Kohlenwasserstoffe (Benzol, Toluol etc.). Geeignete Basen sind beispielsweise tertiäre Amine, wie Triethylamin, Triethanolamin oder N-Methylmorpholin, Alkalimetallhydroxide, wie Natronlauge oder Kalilauge oder Alkalimetallalkoholate, wie Natriummethylat oder Kaliummethylat. Dieser erste Reaktionsschritt wird üblicherweise bei einer Reaktionstemperatur von -20°C bis 100°C durchgeführt, wobei eine Temperatur von 0°C bis 30°C bevorzugt angewendet wird.

In der zweiten Stufe des Verfahrens werden die erhaltenen Diacylamine mit der erwünschten L-Aminosäure zur Reaktion gebracht. Auch diese Stufe wird vorzugsweise in einem inerten Lösungsmittel in Gegenwart basischer Katalysatoren durchgeführt. Als Lösungsmittel können die gleichen Solventien wie in dem ersten Verfahrensschritt verwendet werden. Es ist aber andererseits auch möglich, diese Reaktion in niederen Ketonen (Aceton, Methylethylketon, Methylisobutylketon etc.) als Lösungsmitteln durchzuführen. Als basische Katalysatoren verwendet man für diesen Verfahrensschritt vorzugsweise eine der oben genannten tert. Amine. Die Reaktionstemperatur beträgt bei diesem Verfahrensschritt beispielsweise -20°C bis 100°C.

Weitere Gegenstände der Erfindung sind auch die Verwendung der erfindungsgemäßen Verbindungen in einem Verfahren zur Behandlung des menschlichen oder tierischen Körpers sowie Arzneimittel, welche die erfindungsgemäßen Verbindungen enthalten.

Zum Gegenstand der Erfindung gehören auch Salze der Verbindungen der Formel (I). Dabei handelt es sich in erster Linie um üblicherweise pharmazeutisch verwendbare, nicht-toxische Salze, z. B. Alkalimetall- oder Ammoniumsalze.

Die Verbindungen der vorliegenden Erfindung weisen wertvolle pharmakologische Eigenschaften insbesondere eine ausgeprägte, die Abwehr steigernde Wirkung auf. Es wurde gefunden, daß die Verbindungen der vorliegenden Erfindung die Antikörpersynthese des Immunsystems steigern und darüber hinaus die unspezifische wirtseigene Abwehr verstärken. Dies kann anhand entsprechender Versuchsanordnungen gezeigt werden.

Die pharmazeutischen Präparate und Zubereitungen der vorliegenden Erfindung sind vorzugsweise Tabletten oder Gelatinekapseln, welche die Wirkstoffe zusammen mit Verdünnungsmitteln, z. B. Laktose, Dextrose, Saccharose, Mannit, Sorbit, Cellulose und/oder Schmiermitteln, z. B. Kieselerde, Talk, Stearinsäure oder Salze davon, wie Magnesium- oder Calciumstearat, und/oder Polyethylenglykol, enthalten. Tabletten enthalten ebenfalls Bindemittel, wie z. B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z. B. Stärken, Agar, Alginsäure oder ein Salz davon, wie Natriumalginat und/oder Brausemischungen, oder Adsorptionsmittel, Farbstoffe, Geschmackstoffe und Süssmittel. Injizierbare Präparate sind vorzugsweise

isotonische wäßrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren hergestellt und enthalten von etwa 0,1 % bis etwa 75 %, insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als abwehrsteigernde und immunpotenzierende Mittel zur Behandlung von chronischen und akuten Infektionen (z. B. bakterielle, virale und parasitäre) und malignen Tumoren verwendet werden. Sie können ebenfalls als Adjuvantien bei der Vakzinierung, bei der Stimulierung von Phagocytose, bei der Dysregulation des Abwehr- und Immunsystems verwendet werden.

Die erfindungsgemäßen Verbindungen können als Adjuvantien eingesetzt werden. Sie sind besonders wirksam in Verbindung mit Squalen oder Squalan.

Die nachfolgenden Beispiele erläutern die vorstehend beschriebene Erfindung, sie sollen jedoch diese in ihrem Umfange in keiner Weise einschränken.

Zur Untersuchung der antimikrobiellen Aktivität wurden bei 20 NMRI-Mäusen je 500 µg der Substanz des Beispiels 1 in 1 % Squalan-in-Wasser-Lösung injiziert. Zur Kontrolle wurden 20 Tiere lediglich mit Kochsalzlösung behandelt. Nach einer Woche wurden alle Tiere mit einem Aerosol von mäusepathogenen Influenza-Viren infiziert. Während alle Kontrolltiere (Kochsalz) starben, überlebten 80 % der Tiere, die mit der Titelsubstanz behandelt wurden.

Die immunkompetenten Zellen der mit dieser Substanz behandelten Tiere zeigten erhöhte Aktivität.

**Beispiel 1**

N-[(1,2:5,6-Di-0-isopropyliden-α-D-glucofuranos-30-yl)-acetyl]-glycyl-D-glutaminsäure-dimethylester

1 mol [(1,2:5,6-Di-0-isopropyliden-3-0-carboxymethyl-α-D-glucofuranose J. Heterocycl. Chem. 19, 981, (1982)] = 318 mg, in 2 ml Ethylacetat wird mit 1 mM Triethylamin und 1.1 mM = 312 mg N-phenyl-2,6-dichlorbenzimidoyl-chlorid versetzt. Nach 4 Stunden Einwirkung wird die erhaltene Substanz mit Wasser gewaschen und dann das Ethylacetat abgedampft, der Rückstand in 5 ml Isopropanol gelöst und mit 1 mM Glycyl-D-glutaminsäuredimethylester in 5 ml Isopropanol betropft. Nach 4 Stunden Reaktion ist das Isopropanol verdampft. Es entsteht eine ölige Substanz. Diese wird über Säulenchromatographie (Silikagel, Lösungsmittel Benzol-Aceton 6 : 1) gereinigt.

So erhält man 436 mg (82 % Ausbeute) der Titelsubstanz. Spektroskopische Prüfungen zeigen den Erfolg der Synthese.

$$[\alpha]_D^{25} = -23{,}58° \ (c = 0.6, \text{Aceton})$$

NMR (in $CDCl_3$): (δ) 1.31 (3H), 1.35 (3H), 1.40 (3H), 1.47 (3H) 3.67 (3H), 3.73 (3H), 3.85 – 4.40 (9H, multiplet), 4.59 (2H, multiplet), 5.92 (1H, doublet), 7.02 (1H, doublet), 8.20 (1H, triplet).
MS : m/z = 532.

**Beispiel 2** (= Substanz in Beispiel 1)

340 mg (1 mM) 1,2 : 5,6-Di-0-isopropyliden-3-0-carboxymethyl-D-glucofuranose-natrium-Salz wird in 3 ml Aceton gelöst und die Lösung auf -10°C gekühlt.

Es werden 0,15 ml Triethylamin hinzugefügt. Unter intensivem Rühren werden 0,11 ml Ethylchloroformat zugetropft. Nach 15 Min. tropft man langsam die Lösung von 1 mM Glycyl-D-glutaminsäure-dimethylester (aus 232 mg Peptidester-hydrochlorid und 0,14 ml Triethylamin) in 4 ml Aceton hinzu. Man rührt die Mischung noch 0,5 Stunden unter Kühlung und 4 Stunden bei Raumtemperatur. Das Reaktionsprodukt wird analog Beispiel 1 gereinigt. Man erhält 315 mg (59 %) reiner Substanz.

**Beispiel 3**

390 mg ( 1 mM) N-[( 1,2 : 5,6-Diisopropyliden-glucofuranosyl3-0-)-acetyl]-L-alanin werden in Aceton gelöst und auf einem Eisbad gekühlt. Danach werden 0,15 ml Triethylamin und 290 mg N-Phenyl-2,6-dichlor-benzimidoyl-chlorid zugegeben. Die Reaktionsmischung wird während eines Tages bei Raumtemperatur gerührt. Das Aceton wird verdampft, der Rückstand in 5 ml Essigester gelöst und mit Wasser gewaschen. Anschließend wird die Esterphase getrocknet und am

Rotationsverdampfer eingeengt. Der Rückstand wird mit 1 mM D-Glutaminsäure-dinatrium-Salz in 4 ml Dimethylformarmid 2 Stunden umgesetzt. Danach wird die Reaktionslösung bei 35°C am Rotationsverdampfer stark eingeengt. Das ölige Produkt wird in Essigester gelöst und mit Wasser extrahiert. Die wäßrige Phase wird mit Essigester bedeckt und in der Kälte mit 0,6 N Zitronensäure versetzt. Die organische Phase wird abgetrennt und über $MgSO_4$ getrocknet. Nach dem Verdampfen erhält man 425 mg (82 %) N-[-N-(1,2 : 5,6-Di-0-isopropyliden-glucofuranosyl-3-0-)-acetyl]-L-alanyl-D-glutaminsäure als weißen Schaum.

$[\alpha]_D^{20}$ = -35,80° (c = 0,4 in Aceton)

In analoger Weise erhält man N-[-N-(1,2 : 5,6-Di-0-isopropyliden-glucofuranosyl-3-0)-acetylglycyl]-D-glutaminsäure mit 79 % der Ausbeute.

**Beispiel 4**

N-[(1,2 : 5,6-Di-isopropyliden-glucofuranosyl-3-0-)-acetyl]N-(2,6-dichlorbenzoyl)-anilin.

Zu einer Lösung aus 680 mg (2 mM) Natrium (1,2 : 5,6-Diisopropyliden-glucofuranosyl-3-0-)-acetat (J. Heterocyclic Chem., 19, 981 (1982)) in 5 ml Essigester und 0,5 ml Wasser werden 595 mg (2,1 mM) N-Phenyl-(2,6-di-chlorbenzimidoyl)-chlorid gegeben und die Mischung 4 Stunden lang gerührt. Danach wird die Wasserschicht abgetrennt und die organische Lösung über $Na_2SO_4$ getrocknet. Das Lösungsmittel wird im Rotationsverdampfer entfernt. Man erhält 1,114 g eines weißen Schaums, der durch Zugabe von etwas Isopropanol kristallisiert.

| | |
|---|---|
| NMR (in $CDCl_3$): (δ) | 1.28 - 1.50 m (12 H, 4x$CH_3$) |
| | 3.60 - 4.24 m (6 H, CH, $CH_2$) |
| | 4.40 - 4.54 m (1H, CH-O-) |
| | 4.84 d (1H, CH-O-) |
| | 5.96 d (1H,-O-CH-O-) |
| | 7.12 - 7.68 m (8H, arom. CH) |

Die Substanz wird ebenfalls als Ausgangsprodukt für die erfindungsgemäßen Verbindungen eingesetzt.

**Beispiel 5**

N-[(1,2 : 5,6-Di-0-isopropyliden-glucofuranosyl-3-0-)acetyl]-L-aminosäure.

Zu 566 mg (1 mM) N-[(1,2 : 5,6-Di-isopropyliden-glucofuranosyl-3-0-)-acetyl]-N-(2,6-dichlorbenzoyl)-anilin in 2 ml Dimethylformamid fügt man bei Raumtemperatur 1 mM L-Amino-säure in Salzform (Na, K, oder Triethylamin) in 2 ml Dimethylformamid. Nach 4 - 8 Stunden wird Dimethylformamid bei 35°C am Rotationsverdampfer eingedampft und der Rückstand in 1 ml Wasser gelöst. Das Reaktionsprodukt wird unter Kühlung in Essigester bis zu einem pH-Wert von 2 angesäuert, die organische Phase abgetrennt und getrocknet. Nach Eindampfen der Lösung erhält man Diisopropylidenglucofuranosyl-acetyl-aminosäure als öligen Sirup oder weißen Schaum in 80 - 90 %iger Ausbeute.
Man erhält so:

N-[(1,2 : 5,6-diisopropyliden-glucofuranosyl-3-0-)-acetyl]glycin,
N- [(1,2 : 5,6-diisopropyliden-glucofuranosyl-3-0-)-acetyl]L-alanin,
N-[(1,2 : 5,6-diisopropyliden-glucofuranosyl-3-0-)-acetyl]L-Phenylalanin

als Ausgangsprodukte für die erfindungsgemäßen Verbindungen.

**Patentansprüche:**

1. Zuckerhaltige Dipeptide der Formel (I)

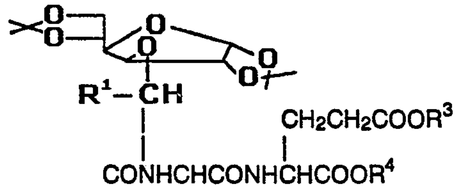

(I)

in der

$R^1$ – Wasserstoff oder eine $C_1$-$C_6$-Alkylgruppe
-HN-CH-CO- – Glycin oder einen natürlichen L-Aminosäurerest
|
$R^2$
$R^3$ – Wasserstoff oder eine $C_1$-$C_6$-Alkylgruppe und
$R^4$ – Wasserstoff oder eine $C_1$-$C_6$-Alkylgruppe

bedeuten, und der Glutamylrest in der D-Form vorliegt,

2. Zuckerhaltige Dipeptide nach Anspruch 1, dadurch gekennzeichnet, daß $R^1$, $R^3$ und $R^4$ gleich oder verschieden sind und für Wasserstoff oder eine $C_1$-$C_4$Alkylgruppe stehen.

3. Zuckerhaltige Dipeptide nach Anspruch 1, dadurch gekennzeichnet, daß $R^1$, $R^3$ und $R^4$ gleich oder verschieden sind und für Wasserstoff, Methyl oder Ethyl stehen.

4. Zuckerhaltige Dipeptide nach den Ansprüchen 1 bis 3, dadurch gekennzeichnet, daß

-HN-CH-CO
|
$R^2$

in Formel I sich von Glycin, L-Alanin, L-Phenylalanin, L-Serin, L-Cystein, L-Lysin, L-Histidin, L-Valin, L-Leucin, L-Isoleucin, L-Tryptophan, L-Asparagin, L-Threonin, L-Tyrosin, L-Methionin, L-Ornithin, L-Arginin, L-Prolin oder L-Hydroxyprolin ableitet.

5. Zuckerhaltige Dipeptide nach den Ansprüchen 1 bis 3, dadurch gekennzeichnet, daß

-HN-CH-CO
|
$R^2$

in Formel (I) sich von Glycin, L-Alanin, L-Phenylalanin, L-Serin oder L-Threonin ableitet.

6. Verfahren zur Herstellung von zuckerhaltigen Dipeptiden der Formel (I)

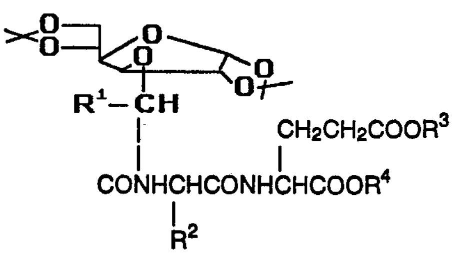

(I)

in der

$R^1$ – Wasserstoff oder eine $C_1$-$C_6$-Alkylgruppe
-HN-CH-CO- – Glycin oder einen natürlichen L-Aminosäurerest
|
$R^2$
$R^3$ – Wasserstoff oder eine $C_1$-$C_6$-Alkylgruppe und
$R^4$ – Wasserstoff oder eine $C_1$-$C_6$-Alkylgruppe

bedeuten, und der Glutamylrest in der D-Form vorliegt, dadurch gekennzeichnet, daß man D-Glucofuranosederivate der Formel (II),

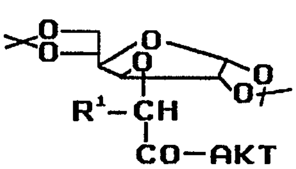

(II)

in der $R^1$ die oben angegebene Bedeutung hat und AKT für eine leicht auswechselbare für Acylierungsreaktionen bekannte Gruppe steht, mit Dipeptidfragmenten der Formel (III)

$$\begin{array}{l} \quad R^2 \qquad CH_2CH_2COOR^3 \\ \quad | \qquad\quad | \\ H_2N\text{-}CHCONHCHCOOR^4 \end{array} \qquad \text{(II)}$$

in der $R^2$, $R^3$ und $R^4$ die oben angegebene Bedeutung haben, zur Reaktion bringt und die Schutzgruppen in den Verbindungen der Formel (I) gegebenenfalls abspaltet.

7. Verfahren zur Herstellung zuckerhaltiger Dipeptide der Formel (I) gemäß Anspruch 6, dadurch gekennzeichnet, daß man Verbindungen der Formel (II) in einem ersten Schritt mit einer natürlichen L-Aminosäure umsetzt, anschließend aktiviert und danach mit D-Glutaminsäure umsetzt.

8. Verfahren zur Herstellung zuckerhaltiger Dipeptide der Formel (I) gemäß Anspruch 6, dadurch gekennzeichnet, daß man Verbindungen der Formel (II) mit einem Carbonsäureimidchlorid der Formel (IV)

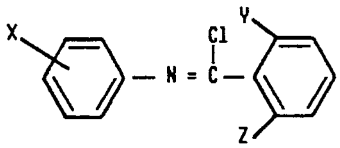

(IV)

in der

X Wasserstoff, $C_1$-$C_4$-Alkyl, Fluor, Chlor oder $NO_2$ bedeutet,
Y Halogen oder $NO_2$ darstellt und
Z die gleiche Bedeutung wie Y besitzt,

umsetzt, das erhaltene Diacylamin mit der gewünschten L-Aminosäure zur Reaktion bringt, die so erhaltene Verbindung erneut mit einem Carbonsäureimidchlorid der Formel (IV) umsetzt und dieses Diacylamin mit D-Glutaminsäure zum Endprodukt der Formel (I) reagieren läßt.

9. Zuckerhaltige Dipeptide der Formel (I)

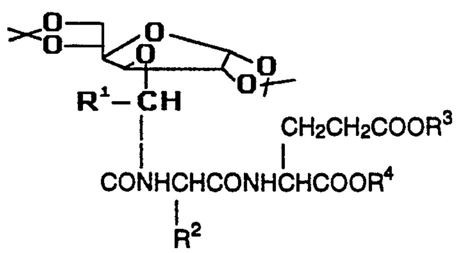

(I)

in der

| | |
|---|---|
| $R^1$ | – Wasserstoff oder eine $C_1$-$C_6$-Alkylgruppe |
| -HN-CH-CO- (mit $R^2$ am CH) | – Glycin oder einen natürlichen L-Aminosäurerest |
| $R^3$ | – Wasserstoff oder eine $C_1$-$C_6$-Alkylgruppe und |
| $R^4$ | – Wasserstoff oder eine $C_1$-$C_6$-Alkylgruppe |

bedeuten, und der Glutamylrest in der D-Form vorliegt, zur Verwendung in einem Verfahren zur therapeutischen Behandlung des menschlichen oder tierischen Körpers.

10. Arzneimittel, enthaltend zuckerhaltige Dipeptide der Formel (I)

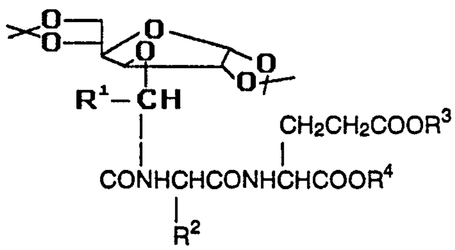

(I)

in der

| | |
|---|---|
| $R^1$ | – Wasserstoff oder eine $C_1$-$C_6$-Alkylgruppe |
| -HN-CH-CO- <br>     \| <br>     $R^2$ | – Glycin oder einen natürlichen L-Aminosäurerest |
| $R^3$ | – Wasserstoff oder eine $C_1$-$C_6$-Alkylgruppe und |
| $R^4$ | – Wasserstoff oder eine $C_1$-$C_6$-Alkylgruppe |

bedeuten, und der Glutamylrest in der D-Form vorliegt,

11. Arzneimittel gemäß Anspruch 10, enthaltend ein Adjuvanz oder ein Gemisch von Adjuvantien.

12. Arzneimittel gemäß Anspruch 10, enthaltend Squalen oder Squalan.

**Claims**

1. Sugar-containing dipeptides having the formula (I)

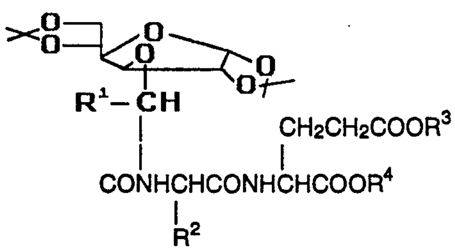

(I)

wherein

| | |
|---|---|
| $R^1$ | represents hydrogen or a $C_1$-$C_6$ alkyl group, |
| -HN-CH-CO- <br>     \| <br>     $R^2$ | represents glycine or a natural L-amino acid residue, |
| $R^3$ | represents hydrogen or a $C_1$-$C_6$ alkyl group, and |
| $R^4$ | represents hydrogen or a $C_1$-$C_6$ alkyl group, |

and the glutamyl residue is present in the D-form.

2. Sugar-containing dipeptides according to claim 1, characterized in that $R^1$, $R^3$ and $R^4$ are same or different and each represent hydrogen or a $C_1$-$C_4$ alkyl group.

3. Sugar-containing dipeptides according to claim 1, characterized in that $R^1$, $R^3$ and $R^4$ are same or different and each represent hydrogen, methyl or ethyl.

4. Sugar-containing dipeptides according to claims 1 to 3, characterized in that

-HN-CH-CO-<br>
    |<br>
    $R^2$

in formula I is derived from glycine, L-alanine, L-phenylalanine, L-serine, L-cysteine, L-lysine, L-histidine, L-valine, L-leucine, L-isoleucine, L-tryptophane, L-asparagine, L-threonine, L-tyrosine, L-methionine, L-ornithine, L-arginine, L-proline or L-hydroxyproline.

5. Sugar-containing dipeptides according to claims 1 to 3, characterized in that

-HN-CH-CO
  |
  $R^2$

in formula I is derived from glycine, L-alanine, L-phenylalanine, L-serine or L-threonine.

6. A process for preparing sugar-containing dipeptides having the formula (I)

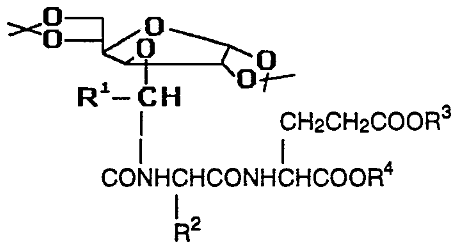

(I)

wherein

$R^1$ represents hydrogen or a $C_1$-$C_6$ alkyl group,

-HN-CH-CO- (with $R^2$ on CH) represents glycine or a natural L-amino acid residue,

$R^3$ represents hydrogen or a $C_1$-$C_6$ alkyl group, and

$R^4$ represents hydrogen or a $C_1$-$C_6$ alkyl group,

and the glutamyl residue is present in the D-form, characterized in that D-glucofuranose derivatives having the formula (II)

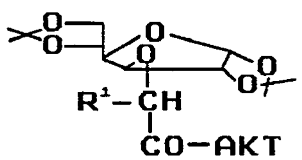

(II)

wherein

$R^1$ as defined above and

AKT represents a readily exchangeable group known for acylating reactions, are reacted with dipeptide fragments having the formula (III)

$R^2$ $CH_2CH_2COOR^3$
  |  |
$H_2N$-CHONHCHCOOR$^4$ (III)

wherein

$R^2$, $R^3$ and $R^4$ are as defined above,

and optionally the protective groups in the compounds having the formula (I) are removed.

7. The process for preparing sugar-containing dipeptides having the formula (I) according to claim 6, characterized in that in a first step compounds having the formula (II) are reacted with a natural L-amino acid, are subsequently activated and thereafter reacted with D-glutamic acid.

8. The process for preparing sugar-containing dipeptides having the formula (I) according to claim 6, characterized in that in a first step compounds having the formula (II) are reacted with a carboxylic acid imide chloride having the formula (IV)

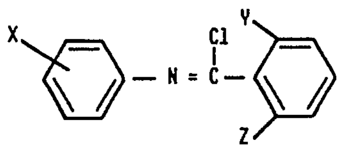

(IV)

wherein

X represents hydrogen, $C_1$-$C_4$ alkyl, fluorine, chlorine or $NO_2$,
Y represents halogen or $NO_2$ and
Z has the same meaning as Y,

the diacylamine obtained is reacted with the desired amino acid, the compound thus obtained is again reacted with a carboxylic acid imide chloride having the formula (IV), and the obtained diacylamine is allowed to react with D-glutamic acid to give the final product having the formula (I).

9. Sugar-containing dipeptides having the formula (I)

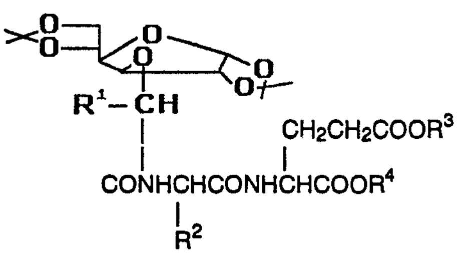

(I)

wherein

| | |
|---|---|
| $R^1$ | represents hydrogen or a $C_1$-$C_6$ alkyl group, |
| -HN-CH-CO-<br>\|<br>$R^2$ | represents glycine or a natural L-amino acid residue, |
| $R^3$ | represents hydrogen or a $C_1$-$C_6$ alkyl group, and |
| $R^4$ | represents hydrogen or a $C_1$-$C_6$ alkyl group, |

and the glutamyl residue is present in the D-form for use in a process for the therapeutic treatment of a human or animal body.

10. A medicament which contains sugar-containing dipeptides having the formula (I)

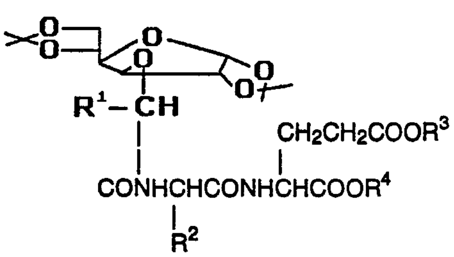

(I)

wherein

| | |
|---|---|
| $R^1$ | represents hydrogen or $C_1$-$C_6$ alkyl group, |
| -HN-CH-CO-<br>\|<br>$R^2$ | represents glycine or a natural L-amino acid residue, |
| $R^3$ | represents hydrogen or a $C_1$-$C_6$ alkyl group, and |
| $R^4$ | represents hydrogen or a $C_1$-$C_6$ alkyl group, |

and the glutamyl residue is present in the D-form.

11. The medicament according to claim 10, containing an adjuvant or a mixture of adjuvants.

12. The medicament according to claim 10, containing squalene or squalane.

## Revendications

1. Dipeptides contenant des sucres, de formule (I):

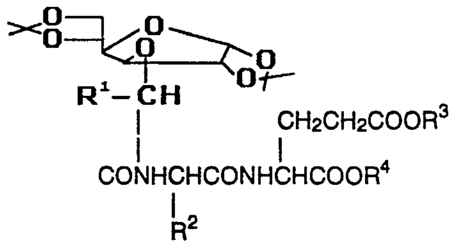

(I)

dans laquelle:

| | |
|---|---|
| $R^1$ | représente un atome d'hydrogène ou un groupe alkyle en $C_1$ à $C_6$, |
| -HN-CH-CO-<br>$\quad$ \|<br>$\quad R^2$ | représente un groupe glycine ou un reste L-amino-acide naturel, |
| $R^3$ | représente un atome d'hydrogène ou un groupe alkyle en $C_1$ à $C_6$, et |
| $R^4$ | représente un atome d'hydrogène ou un groupe alkyle en $C_1$ à $C_6$, |

et le reste glutamyle est présent sous forme D.

2. Dipeptide contenant des sucres selon la revendication 1, caractérisés en ce que $R^1$, $R^3$ et $R^4$ sont identiques ou différents et représentent un atome d'hydrogène ou un groupe alkyle en $C_1$ à $C_4$.

3. Dipeptide contenant des sucres,selon la revendication 1 , caractérisés en ce que $R^1$, $R^3$ et $R^4$ sont identiques ou différents et représentent un atome d'hydrogène ou un reste méthyle ou éthyle.

4. Dipeptides contenant des sucres selon les revendications 1 à 3, caractérisés en ce que, dans la formule I,

-HN-CH-CO
$\quad$ |
$\quad R^2$

dérive de la glycine, de la L-alanine, de L-phényl-alanine, de la Lsérine, de la L-cystéine, de la L-lysine, de la L-histidine, de la Lvaline, de la L-leucine, de la L-isoleucine, du L-tryptophane, de la L-asparagine, de la L-thréonine, de la L-tyrosine, de la L-méthionine, de la L-ornithine, de la L-arginine, de la L-proline ou de la L-hydroxyproline.

5. Dipeptides contenant des sucres selon les revendications 1 à 3, caractérisés en ce que, dans la formule (I),

-HN-CH-CO-
$\quad$ |
$\quad R^2$

dérive de la glycine, de la L-alanine, de la L-phényl-alanine, de la Lsérine ou de la L-thréonine.

6. Procédé pour préparer des dipeptides contenant des sucres, de formule (I)

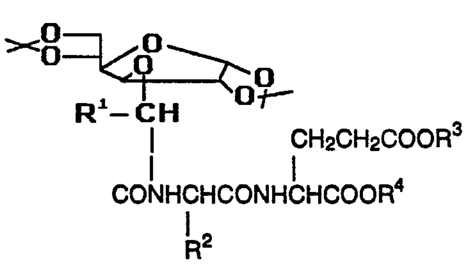

(I)

dans laquelle:

| | |
|---|---|
| $R^1$ | représente un atome d'hydrogène ou un groupe alkyle en $C_1$ à $C_6$, |

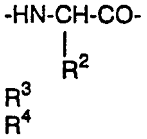 représente la glycine ou un reste de L-amino-acide naturel,

$R^3$ représente un atome d'hydrogène ou un groupe alkyle en $C_1$ à $C_6$, et

$R^4$ représente un atome d'hydrogène ou un groupe alkyle en $C_1$ à $C_6$,

et le groupe glutamyle est présent sous forme D, procédé caractérisé en ce qu'on fait réagir des dérivés du D-glucofurannose, de formule (II)

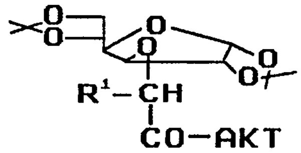

(II)

(dans laquelle $R^1$ à le sens indiqué ci-dessus et AKT représente un groupe connu, facilement échangeable, pour des réactions d'acylation) avec des fragments dipeptides de formule (III)

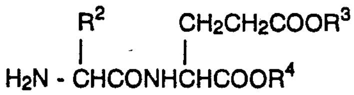

(III)

(dans laquelle $R^2$, $R^3$ et $R^4$ ont le sens indiqué ci-dessus) et l'on élimino éventuellement les groupes protecteurs présents dans les composés de formule (I).

7. Procédé de préparation de dipeptides contenant des sucres, de formule (1) selon la revendication 6, caractérisé en ce que, dans une première étape, on fait réagir des composés de formule (II) avec un L-amino-acide naturel, puis on active et l'on fait ensuite réagir avec l'acide D-glutamique.

8. Procédé de préparation de dipeptides contenant des sucres, de formule (I) selon la revendication 6, caractérisé en ce qu'on fait réagir des composés de formule (II) avec un imidochlorure d'acide carboxylique de formule (IV):

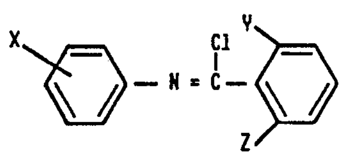

(IV)

dans laquelle:

- X représente un atome d'hydrogène,un groupe alkyle en $C_1$ à $C_4$, un atome de fluor, un atome de chlore ou un groupe $NO_2$,
- Y représente un atome d'halogène ou un groupe $NO_2$
- Z a la même signification qu'Y on fait réagir la diacylamine ainsi obtenue avec le L-amino-acide voulu, on fait à nouveau réagir le composé ainsi obtenu avec un imidochlorure d'acide carboxylique de formule (IV), et l'on fait réagir cette diacylamine avec l'acide D-glutamique pour obtenir le produit final de formule (I).

9. Dipeptides contenant des sucres, de formule (I)

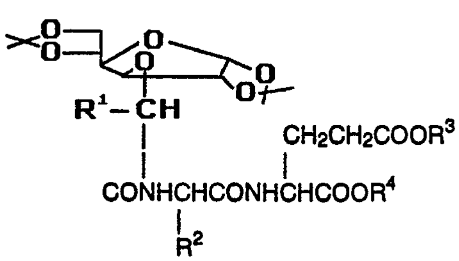

(I)

dans laquelle:

$R^1$ représente un atome d'hydrogène ou un groupe alkyle en $C_1$ à $C_6$,

| | |
|---|---|
| -HN-CH-CO-<br>\|<br>$R^2$ | représente la glycine ou un reste de L-amino-acide naturel, |
| $R^3$ | représente un atome d'hydrogène ou un groupe alkyle en $C_1$ à $C_6$, et |
| $R^4$ | représente un atome d'hydrogène ou un groupe alkyle en $C_1$ à $C_6$, |

et le reste glutamyle est présent sous forme D) pour servir dans un procédé de traitement thérapeutique du corps humain ou animal.

10. Médicament, contenant des dipeptides, contenant des sucres, de formule (I)

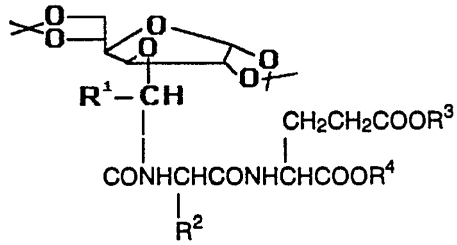

(I)

dans laquelle:

| | |
|---|---|
| $R^1$ | représente un atome d'hydrogène ou un groupe alkyle en $C_1$ à $C_6$, |
| -HN-CH-CO-<br>\|<br>$R^2$ | représente la glycine ou un reste de L-amino-acide naturel, |
| $R^3$ | représente un atome d'hydrogène ou un groupe alkyle en $C_1$ à $C_6$, et |
| $R^4$ | représente un atome d'hydrogène ou un groupe alkyle en $C_1$ à $C_6$, |

et le reste glutamyle est présent sous forme D).

11. Médicament selon la revendication 10, contenant un adjuvant ou un mélange d'adjuvants.

12. Médicament selon la revendication 10, contenant du squalène ou du squalane.